# EUROPEAN PATENT APPLICATION

(11) **EP 3 151 008 A1**
(43) Date of publication of application: **05.04.2017**
(21) Application number: 15800622.1
(22) Date of filing: 16.03.2015
(51) Int. Cl.: G01N 33/68, C12Q 1/04, G01N 33/50

(54) **PANCREATIC CANCER DETERMINATION METHOD**

(30) Priority: 26.05.2014 JP 2014108454
(71) Applicant: Olympus Corporation, Hachioji-shi Tokyo 192-8507 (JP)
(72) Inventor: NAKATA, Mari, Hachioji-shi Tokyo 192-8507 (JP)
(74) Representative: Schicker, Silvia
(86) International application number: PCT/JP2015/057673
(87) International publication number: WO 2015/182218

(57) **Abstract**

A pancreatic cancer determination method of the present invention, which determines pancreatic cancer in a subject, includes: a first process of measuring the concentration of a first marker in the duodenal juice of the subject; a second process of measuring the concentration of a second marker in a blood sample of the subject; and a third process of determining at least one of the presence of pancreatic cancer and the stage of progression of the pancreatic cancer in the subject according to whether the concentration of the first marker is greater than or equal to a first cutoff value and whether the concentration of the second marker is greater than or equal to a second cutoff value.

## Description

### [Technical Field]

The present invention relates to a pancreatic cancer determination method. Priority is claimed on Japanese Patent Application No. 2014-108454, filed May 26, 2014, the content of which is incorporated herein by reference.

### [Background Art]

In the related art, the presence or the like of cancer has been frequently determined by measuring tumor markers included in various samples collected from subjects of cancer patients or the like.

Various attempts have been made even for pancreatic cancer which is a type of cancer. Patent Document 1 describes that the type of pancreatic disease is determined based on the concentration of VEGF-A, PGE2, or VEGF-C in the pancreatic juice. Patent Document 2 describes that pancreatic cancer is diagnosed based on the concentration of REG4 and CA19-9 in the blood.

### [Citation List]

### [Patent Document]

[Patent Document 1] United States Patent Application, Publication No. 2012/0231480
[Patent Document 2] United States Patent Application, Publication No.

### [Summary of Invention]

### [Technical Problem]

However, in the techniques described in Patent Documents 1 and 2, it is difficult to determine the presence of pancreatic cancer and the stage of progression of pancreatic cancer.

In view of the above-described problem, an object of the present invention is to provide a pancreatic cancer determination method which is capable of easily determining the pancreatic cancer.

### [Solution to Problem]

According to a first aspect of the present invention, a pancreatic cancer determination method, which determines pancreatic cancer in a subject, includes: a first process of measuring a concentration of a first marker in the duodenal juice of the subject; a second process of measuring a concentration of a second marker in a blood sample of the subject; and a third process of determining at least one of the presence of pancreatic cancer and the stage of progression of the pancreatic cancer in the subject based on whether the concentration of the first marker is greater than or equal to a first cutoff value and whether the concentration of the second marker is greater than or equal to a second cutoff value.

According to a second aspect of the present invention, the stage of progression of the pancreatic cancer may be determined to be an early stage in the third process when the concentration of the first marker is greater than or equal to the first cutoff value and the concentration of the second marker is less than the second cutoff value.

According to a third aspect of the present invention, the stage of progression of the pancreatic cancer may be determined to be a middle stage in the third process when the concentration of the first marker is greater than or equal to the first cutoff value and the concentration of the second marker is greater than or equal to the second cutoff value.

According to a fourth aspect of the present invention, the stage of progression of the pancreatic cancer may be determined to be a stage more progressed than the middle stage in the third process when the concentration of the first marker is less than the first cutoff value and the concentration of the second marker is greater than or equal to the second cutoff value.

According to a fifth aspect of the present invention, the subject may be determined not to have pancreatic cancer when the concentration of the first marker is less than the first cutoff value and the concentration of the second marker is less than the second cutoff value, and the subject may be determined to have pancreatic cancer in all the other cases in the third process.

According to a sixth aspect of the present invention, the first marker may be S100P.

According to a seventh aspect of the present invention, the second marker may be CA19-9.

### [Advantageous Effects of Invention]

According to each of the aspects described above, it is possible to provide a pancreatic cancer determination method which is capable of easily determining the pancreatic cancer.

### [Brief Description of Drawings]

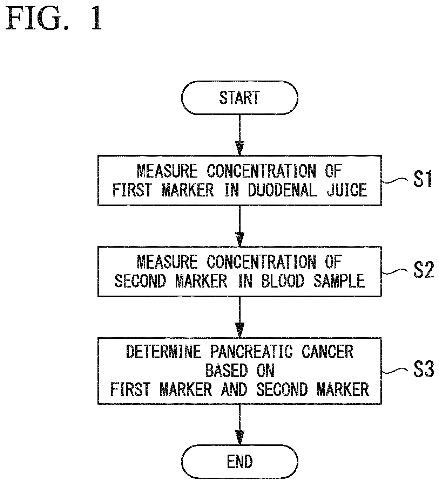

FIG. 1 is a flowchart showing a flow of a pancreatic cancer determination method of the present invention.

### [Description of Embodiments]

First, the outline of a method of determining pancreatic cancer (hereinafter, simply referred to as "a pancreatic cancer determination method") of the present invention will be described and then a first embodiment will be described.

FIG. 1 is a flowchart showing a flow of the pancreatic cancer determination method of the present invention. The pancreatic cancer determination method includes Step S1 of determining the concentration of a first marker in the duodenal juice of a subject or the like; Step S2 of measuring the concentration of a second marker in a blood sample of the same subject or the like; and Step S3 of determining the pancreatic cancer in the subject or the like based on the concentration of the first marker and the concentration of the second marker.

In the first process of Step S1, the duodenal juice of a patient is collected. The duodenal juice is a mixture of body fluids of pancreatic juice discharged from the pancreas, bile discharged from the gallbladder or the liver, and mucus (intestinal juice) secreted from the duodenum. A method of collecting the duodenal juice is not particularly limited, but the duodenal juice can be easily collected by performing suction or the like using an endoscope inserted into a body of a subject or the like. The specimen amount of duodenal juice may be approximately 50 microliters (µl).

In the first process, the concentration of the first marker in the duodenal juice as a mixed body fluid is measured without isolating the pancreatic juice from the duodenal juice. Examples of the first marker include carcinoembryonic antigen (CEA) and S100P which is a kind of S 100 proteins.

In the second process of Step S2, a blood sample of a patient is collected. The specific type (the serum, the plasma, or the like) of the blood sample and the method of collecting the blood sample can be appropriately selected according to the second marker. Examples of the second marker include carbohydrate antigen 19-9 (CA19-9) and the like.

Since the first marker included in the duodenal juice has higher organ specificity than the second marker included in the blood sample, there is an advantage that the first marker is detected even in an early stage of pancreatic cancer. Meanwhile, when stenosis or occlusion of the pancreatic duct occurs as a result of growth of a tumor, the amount of pancreatic juice to be discharged is decreased in some cases. Even in a case where the pancreatic function is degraded due to the progress of pancreatic cancer, the amount of pancreatic juice to be discharged is decreased.

In these cases, since the amount of pancreatic juice in the duodenal juice is decreased, it is difficult to determine the presence of pancreatic cancer and the stage of progression only from the concentration of the first marker included in the duodenal juice.

In the present invention, in consideration of the above-described characteristics of the duodenal juice, the precision of determination related to the pancreatic cancer is improved by means of combining the concentration of the first marker and the concentration of the second marker which is relatively stably detected regardless of the stage of the pancreatic cancer in spite of the inferior organ specificity than that of the first marker.

In the third process of Step S3, at least one of the presence of pancreatic cancer in a subject and the stage of progression of pancreatic cancer in the subject is determined based on the concentration of the first marker and the concentration of the second marker. Specifically, it is determined whether the first marker is positive (+) or negative (-) based on whether the concentration of the first marker is greater than or equal to a first cutoff value. Next, it is determined whether the second marker is positive (+) or negative (-) according to whether the concentration of the second marker is greater than or equal to a second cutoff value.

The first cutoff value and the second cutoff value are individually set for each marker. Further, cutoff values different from each other may be used for the determination of the presence of pancreatic cancer and the determination of the stage of progression of pancreatic cancer.

In a case where the stage of progression of pancreatic cancer is determined according to the pancreatic cancer determination method of the present invention, the stage of progression of pancreatic cancer is determined to be an early stage (stage 0 or I) in a case where the first marker is positive and the second marker is negative (first group). The stage of progression of pancreatic cancer is determined to be a middle stage (stage II) more progressed than the early stage in a case where the first marker is positive and the second marker is positive (second group). The stage of progression of pancreatic cancer is determined to be a terminal stage (stage III or IV) more progressed than the middle stage in a case where the first marker is negative and the second marker is positive (third group).

Note that, as the staging used in the present invention, UICC classification (sixth edition) is used. Further, in a case where both of the first marker and the second marker are negative, this case is treated as undeterminable.

In the present embodiment, in a case where the presence of pancreatic cancer is determined according to the pancreatic cancer determination method of the present invention, it is determined that "the subject has pancreatic cancer" in a case where at least one of the first marker and the second marker is positive. Further, it is determined that "the subject does not have pancreatic cancer" in a case where both of the first marker and the second marker are negative (fourth group). In other words, it is determined that "the subject does not have pancreatic cancer" when the concentration of the first marker is less than the first cutoff value and the concentration of the second marker is less than the second cutoff value, and it is determined that "the subject has pancreatic cancer" in all the other cases.

A first embodiment of the present invention will be described. In the present embodiment, CEA (first cutoff value of 150 ng/ml) is used as the first marker and CA19-9 (second cutoff value of 37 U/ml) is used as the second marker. The determination results of the present embodiment with respect to 68 pancreatic cancer patients are listed in Table 1.
Table 1 shows determination results of the first marker and the second marker which are acquired after classification of 68 pancreatic cancer patients for each stage.

The stage of each patient is decided based on the result of pathological diagnosis in a case of a patient who has undergone an operation and decided according to the result of clinical diagnosis from a doctor in a case of a patient who does not have undergone an operation.

In a case where the determination result related to the stage of progression of pancreatic cancer based on the measurement result of the first marker and the second marker matches the determination result related to the stage of progression of pancreatic cancer based on the result of pathological diagnosis and the result of clinical diagnosis, the numerical values in Table 1 are underlined.

**[Table 1]**

| CLASSIFICATION OF STAGE OF PATIENT BASED ON RESULT OF PATHOLOGICAL DIAGNOSIS AND RESULT OF CLINICAL DIAGNOSIS | | CLASSIFICATION BASED ON MEASUREMENT RESULTS OF CEA AND CEA19-9 | | | | SENSITIVITY (%) WITH RESPECT TO PRESENCE OR ABSENCE OF PANCREATIC CANCER ACCORDING TO DETERMINATION METHOD USING MEASUREMENT RESULTS OF BOTH OF CEA AND CA19-9 | SENSITIVITY (%) WITH RESPECT TO PRESENCE OR ABSENCE OF PANCREATIC CANCER ACCORDING TO DETERMINATION METHOD USING MEASUREMENT RESULT OF ONLY ONE OF CEA AND CA19-9 | |
|---|---|---|---|---|---|---|---|---|
| UICC stage | n | FIRST GROUP (CEA IS POSITIVE AND CEA19-9 IS NEGATIVE) | SECOND GROUP (CEA IS POSITIVE AND CEA19-9 IS POSITIVE) | THIRD GROUP (CEA IS NEGATIVE AND CA19-9 IS POSITIVE) | FOURTH GROUP (CEA IS NEGATIVE AND GA19-9 IS NEGATIVE) | | IN CASE OF USING ONLY MEASUREMENT RESULT OF CEA | IN CASE OF USING ONLY MEASUREMENT RESULT OF CA19-9 |
| EARLY STAGE (Stage 0/1) | 10 | 6 | 1 | 1 | 2 | 80.0 (8/10) | 70.0 | 20.0 |
| MIDDLE STAGE (Stage II) | 53 | 14 | 22 | 11 | 6 | 88.7 (47/53) | 67.9 | 62.3 |
| TERMINAL STAGE (Stage III/IV) | 5 | 2 | 1 | 2 | 0 | 100.0 (5/5) | 60.0 | 60.0 |
| total | 68 | 22 | 24 | 14 | 8 | 88.2 (60/68) | 67.6 | 55.9 |
| HIT RATE | | 27.3 | 91.7 | 14.3 | | | | |

In Table 1, the hit rate in the early-stage patients is 60% (6/10) and the hit rate thereof is 75% (6/8) when the false negativity (fourth group in which the first marker and the second marker are both negative) is excluded. The hit rate in the middle stage patients is 41.5% (22/53) and the hit rate thereof is 46.8% (22/47) when the false negativity is excluded. The hit rate in the terminal stage patients is 40% (2/5), and the false negativity has not been observed.

Further, the hit rates of respective groups from the first group to the third group are 27.3% (6/22) in the case of the first group, 91.7% (22/24) in the case of the second group, and 14.3% (2/14) in the case of the third group.

In Table 1, a concordance rate in a case where the determination result related to the stage of progression of pancreatic cancer based on the measurement result of the first marker and the second marker matches the determination result related to the stage of progression of pancreatic cancer according to the result of pathological diagnosis and the result of clinical diagnosis is 44.1% (30/68). Further, the concordance rate is 50% (30/60) when the false negativity is excluded.

Further, the sensitivities with respect to the presence of pancreatic cancer in the pancreatic cancer determination method of the present embodiment are 80.0% in the case of early-stage patients, 88.7% in the case of middle-stage patients, and 100.0% in the case of terminal-stage patients as listed in Table 1, and the sensitivity in all stages is 80% or greater. The sensitivities with respect to the presence of pancreatic cancer in a case of using only the first marker and in a case of using only the second marker are both 70% or less. That is, the sensitivity with respect to the presence of pancreatic cancer in the pancreatic cancer determination method of the present embodiment is higher than the sensitivities with respect to the presence of pancreatic cancer in a case of using only the first marker and in a case of using only the second marker.

In addition, although data is not shown, the specificity in the same research performed with respect to 29 non-pancreatic cancer patients is 66% and the total proper diagnosis rate is 72.2% (70/97).

As listed in Table 1, in a case of using only the measurement result of CEA, there is a tendency that the sensitivity with respect to the presence of pancreatic cancer is decreased as the stage progresses, but the tendency is not so significant. In a case of using only the measurement result of CA19-9, there is a tendency that the sensitivity with respect to the presence of pancreatic cancer is increased as the stage progresses, but a difference in sensitivity between the middle stage and the terminal stage is not significant. Therefore, in both cases of using only the measurement result of CEA and using only the measurement result of CA19-9, it is difficult to determine the stage of progression. However, it is possible to determine the stage of progression with high precision by performing determination using a combination of these two markers.

It is shown that the presence of pancreatic cancer can be also determined with high precision by combining two markers compared to the case of determining the presence of pancreatic cancer using only one marker.

As described above, the pancreatic cancer determination method of the present embodiment includes the third process of performing determination of pancreatic cancer based on the concentration of the first marker and the second marker, it is possible to determine the stage of progression of pancreatic cancer with high precision and the presence of pancreatic cancer using samples of the duodenal juice and the blood which can be relatively easily obtained.

Next, a second embodiment of the present invention will be described. In the present embodiment, S100P (cutoff value of 50000 pg/ml) is used as the first marker in place of CEA. Table 2 shows the results of performing determination of the present embodiment with respect to 60 pancreatic cancer patients. Table 2 also shows respective positive rates of S100P and CA19-9, similar to Table 1.

Table 2 shows determination results using the first marker and the second marker which are acquired after classification of 60 pancreatic cancer patients for each stage.

The stage of each patient is decided based on the result of pathological diagnosis in a case of a patient who has undergone an operation and decided according to the result of clinical diagnosis from a doctor in a case of a patient who does not have undergone an operation similar to Table 1.

In a case where the determination result related to the stage of progression of pancreatic cancer according to the measurement result of the first marker and the second marker matches the determination result related to the stage of progression of pancreatic cancer according to the result of pathological diagnosis and the result of clinical diagnosis, the numerical values in Table 2 are underlined.

**[Table 2]**

| CLASSIFICATION OF STAGE OF PATIENT BASED ON RESULT OF PATHOLOGICAL DIAGNOSIS AND RESULT OF CLINICAL DIAGNOSIS | | CLASSIFICATION BASED ON MEASUREMENT RESULTS OF S100P AND CA19-9 | | | | SENSITIVITY (%) WITH RESPECT TO PRESENCE OR ABSENCE OF PANCREATIC CANCER ACCORDING TO DETERMINATION METHOD USING MEASUREMENT RESULTS OF BOTH OF S100P AND CA19-9 | SENSITIVITY (%) WITH RESPECT TO PRESENCE OR ABSENCE OF PANCREATIC CANCER ACCORDING TO DETERMINATION METHOD USING MEASUREMENT RESULT USING ONLY ONE OF S100P AND CA19-9 | |
|---|---|---|---|---|---|---|---|---|
| UICC stage | n | FIRST GROUP (S100P IS POSITIVE AND CA19-9 IS NEGATIVE) | SECOND GROUP (S100P IS POSITIVE AND CA19-9 IS POSITIVE) | THIRD GROUP (S100P IS NEGATIVE AND CA19-9 IS POSITIVE) | FOURTH GROUP (S100P IS NEGATIVE AND CA19-9 IS NEGATIVE) | | IN CASE OF^{:} USING ONLY MEASUREMENT RESULT OF S100P | IN CASE OF USING ONLY MEASUREMENT RESULTS OF CA19-9 |
| EARLY STAGE (Stage 0/1) | 9 | 5 | 1 | 1 | 2 | 77.8 (7/9) | 66.7 | 22.2 |
| MIDDLE STAGE (Stage II) | 41 | 8 | 16 | 12 | 5 | 87.8 (36/41) | 58.5 | 68.3 |
| TERMINAL STAGE (Stage III/IV) | 10 | 1 | 3 | 4 | 2 | 80.0 (8/10) | 40.0 | 70.0 |
| total | 60 | 14 | 20 | 17 | 9 | 85.0 (51/60) | 56.7 | 61.7 |
| HIT RATE | | 35.7 | 80.0 | 23.5 | | | | |

In Table 2, the measurement results are obtained from 60 pancreatic cancer patients as subjects, and the hit rate in the early-stage patients is 55.6% (5/9) and the hit rate thereof is 71.4% (5/7) when the false negativity (fourth group in which the first marker and the second marker are both negative) is excluded. The hit rate in the middle stage patients is 39.0% (16/41) and the hit rate thereof is 44.4% (16/36) when the false negativity is excluded. The hit rate in the terminal stage patients is 40% (4/10) and the hit rate is 50% (4/8) when the false negativity is excluded.

Further, the hit rates of respective groups from the first group to the third group are 35.7% (5/14) in the case of the first group, 80.0% (16/20) in the case of the second group, and 23.5% (4/17) in the case of the third group. Therefore, the results are excellent compared to those of the first embodiment.

In Table 2, a concordance rate in a case where the determination result related to the stage of progression of pancreatic cancer based on the measurement result of the first marker and the second marker matches the determination result related to the stage of progression of pancreatic cancer based on the result of pathological diagnosis and the result of clinical diagnosis is 41.7% (25/60). Further, the concordance rate is 49.0% (25/51) when the false negativity is excluded.

The sensitivities with respect to the presence of pancreatic cancer are substantially the same as those of the first embodiment regardless of the stages. In addition, although data is not shown, the specificity in the same research performed with respect to 28 non-pancreatic cancer patients is 75% and the total proper diagnosis rate is 81.8% (72/88).

As described above, even in the second embodiment in which the second marker is changed, it is possible to determine pancreatic cancer with precision which is substantially the same as the precision of the first embodiment.

Hereinbefore, each of the embodiments of the present invention has been described, but the technical scope of the present invention is not limited to the above-described embodiments and various modifications can be added within the range not departing from the scope of the present invention.

For example, in the pancreatic cancer determination method of the present invention, the order of the first process and the second process is not limited and any one of the processes may be performed first.

Further, the cutoff values of the first marker and the second marker are not limited to the values of the above-described embodiments. Therefore, the cutoff values may be set according to accumulated data or the like.

Moreover, even the details of the third process are not limited to the contents of the above-described embodiments. For example, determination of the third process may be performed by providing two or more cutoff values for one or both of the first marker and the second marker such that determination is carried out in three or more stages and the determination results are associated with the subdivided matrix and the stages of progression.

### [Industrial Applicability]

According to each of the above-described embodiments, it is possible to provide a pancreatic cancer determination method which is capable of easily determining the presence of pancreatic cancer.

### [Reference Signs List]

S1: first process
S2: second process
S3: third process

## Claims

1. A pancreatic cancer determination method, which determines pancreatic cancer in a subject, comprising:
a first process of measuring a concentration of a first marker in the duodenal juice of the subject;
a second process of measuring a concentration of a second marker in a blood sample of the subject; and
a third process of determining at least one of the presence of pancreatic cancer and the stage of progression of the pancreatic cancer in the subject based on whether the concentration of the first marker is greater than or equal to a first cutoff value and whether the concentration of the second marker is greater than or equal to a second cutoff value.

2. The pancreatic cancer determination method according to claim 1,
wherein the stage of progression of the pancreatic cancer is determined to be an early stage in the third process when the concentration of the first marker is greater than or equal to the first cutoff value and the concentration of the second marker is less than the second cutoff value.

3. The pancreatic cancer determination method according to claim 1 or 2,
wherein the stage of progression of the pancreatic cancer is determined to be a middle stage in the third process when the concentration of the first marker is greater than or equal to the first cutoff value and the concentration of the second marker is greater than or equal to the second cutoff value.

4. The pancreatic cancer determination method according to claim 3,
wherein the stage of progression of the pancreatic cancer is determined to be a stage more progressed than the middle stage in the third process when the concentration of the first marker is less than the first cutoff value and the concentration of the second marker is greater than or equal to the second cutoff value.

5. The pancreatic cancer determination method according to claim 1,
wherein the subject is determined not to have pancreatic cancer when the concentration of the first marker is less than the first cutoff value and the concentration of the second marker is less than the second cutoff value, and the subject is determined to have pancreatic cancer in all the other cases in the third process.

6. The pancreatic cancer determination method according to any one of claims 1 to 5,
wherein the first marker is S100P.

7. The pancreatic cancer determination method according to any one of claims 1 to 6,
wherein the second marker is CA19-9.
